(19) Europäisches Patentamt
European Patent Office
Office européen des brevets

(11) Publication number: **0 362 002 B1**

(12) # EUROPEAN PATENT SPECIFICATION

(45) Date of publication of patent specification: **26.07.95**

(51) Int. Cl.[6]: **C07K 5/02**, C07K 5/06, C07K 5/08, C07K 5/10, C07K 7/02, C07C 311/50, C07C 311/51, C07C 271/22, C07C 271/20, C07C 237/22, C07C 237/10

(21) Application number: **89402373.8**

(22) Date of filing: **31.08.89**

The file contains technical information submitted after the application was filed and not included in this specification

(54) **HIV protease inhibitors.**

(30) Priority: **01.09.88 EP 88402205**

(43) Date of publication of application:
**04.04.90 Bulletin 90/14**

(45) Publication of the grant of the patent:
**26.07.95 Bulletin 95/30**

(84) Designated Contracting States:
**AT BE CH DE ES FR GB GR IT LI LU NL SE**

(56) References cited:
**EP-A- 0 275 101**
**WO-A-87/04349**

**TETRAHEDRON LETTERS, vol. 29, no. 30, 1988, pages 3687-3690, Pergamon Pressplc; D. SCHIRLIN et al.: "A convenient synthesis of alpha' , beta-diamino-**

**alpha, alpha-difluoroketones, new dipeptide isosteres"**

(73) Proprietor: **MERRELL DOW PHARMACEUTICALS INC.**
**P.O. Box 156300**
**2110 East Galbraith Road**
**Cincinnati**
**Ohio 45215-6300 (US)**

(72) Inventor: **Schirlin, Daniel**
**12, rue Jean Jacques Rousseau**
**Hoenheim**
**F-678000 Bischheim (FR)**
Inventor: **Pelton, John Tom**
**3, rue Murner**
**F-67000 Strasbourg (FR)**
Inventor: **Tarnus, Céline**
**43, rue du 22 Novembre**
**F-67000 Strasbourg (FR)**
Inventor: **Jung, Michel**
**3 Place Echauffour**
**Engwiller**
**F-67350 Pfaffenhoffen (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 362 002 B1

**CHEMICAL ABSTRACTS, vol. 107, no. 25, 21st December 1987, page 368, abstractno. 231968r, Columbus, Ohio, US; I. KATOH et al.: "Inhibition of retroviralprotease activity by an aspartyl proteinase inhibitor",& NATURE (LONDON) 1987, 329(6140), 654-6**

**PROCEEDINGS OF THE NATIONAL ACADEMY OF SCIENCES, vol. 85, no. 18, September1988, pages 6612-6616; S. SEELMEIER et al.: "Human immunodeficiency virus hasan aspartic-type protease that can be inhibited by pepstatin A"**

**BIOCHEMISTRY, vol. 24, no. 8, 9th April 1985, pages 1813-1817, AmericanChemical Society; M.H. GELB et al.: "Fluoro ketone inhibitors of hydrolyticenzymes"**

(74) Representative: **Gillard, Marie-Louise et al**
**Cabinet Beau de Loménie**
**158, rue de l'Université**
**F-75340 Paris Cédex 07 (FR)**

## Description

This invention relates to protease enzyme inhibitors useful for a variety of physiological end-use applications.

Peptide analogs which have protease enzyme inhibiting activity have already been disclosed. In this respect, reference may be made to the following documents :

- EP-A3-0275101 which relates to electrophilic ketone retroamide analogs of certain peptidase substrates having the formula

$R_1NHCHR_2COCF_2CHR_3(NR_bCOXR_a)_nQ$

- WO 87/04349 which discloses a genus of novel peptide analogs which have potent renin-inhibiting activity ;
- Tetrahedron Letters, vol. 29, n°30, 1988, 3687-3690 which discloses a convenient synthesis of $\alpha',\beta$-diamino-$\alpha,\alpha$-difluoroketones useful for the design of serin protease inhibitors.

A new family of protease enzyme inhibitors has now been found.

In its broad aspects, this invention relates to analogs of peptidase substrates in which the nitrogen atom of the scissile amide bond of a partial retropeptide analog of the substrate has been replaced by a difluoromethylene moiety. These peptidase substrate analogs provide specific enzyme inhibitors for a variety of proteases, the inhibition of which exert valuable pharmacological activities and therefore have useful physiological consequences in a variety of disease states.

In its more specific aspects, this invention relates to activated electrophilic ketone retroamide analogs of certain peptidase substrates which are useful in inhibiting serine-, thiol-, carboxylic acid- and metallo-dependent proteolytic enzymes, the inhibition of which will have useful physiological consequences in a variety of disease states.

Still more specifically, this invention relates to activated electrophilic ketone retroamide analogs of peptidase substrates which fall within the following generic groupings characterized according to their active site dependencies. Such generic groupings are:

I. Serine Dependent Enzymes: These include such enzymes such as Elastase (human leukocyte), Cathepsin G, Thrombin, Plasmin, C-1 Esterase, C-3 Convertase, Urokinase, Plasminogen Activator, Acrosin, $\beta$-Lactamase, D-Alanine-D-Alanine Carboxypeptidase, Chymotrypsin, Trypsin and Kallikreins.

II. Thio Dependent Enzymes: Cathepsin B.

III. Carboxylic Acid Dependent Enzymes: These include such specific enzymes as Renin, Pepsin and Cathepsin D.

IV. Metallo Dependent Enzymes: These include Angiotensin Converting Enzyme, Enkephalinase, Pseudomonas Elastase and Leucine Aminopeptidase.

The contemplated peptidase inhibitors of the foregoing enzymes are compounds of the formula

$R_1NHCHR_2C(O)CF_2CHR_3NHC(O)X'$   B

and the hydrates, isosteres or the pharmaceutically acceptable salts thereof, wherein:

- $R_1$ is hydrogen, an $\alpha$-amino protecting group of Groups K' and K, an $\alpha$-amino acid or a peptide comprised of 2 to 8 $\alpha$-amino acid units, the terminal amine of said $\alpha$-amino acid and peptide optionally bearing a protecting group of Groups K' and K,
- $R_2$ is a side chain of the $\alpha$-amino acid, a moiety of Group J or CHM which are responsible for directing the inhibitor to the active site of the enzyme,
- $R_3$ is H, $C_{1-7}$ alkyl, phenyl, benzyl, phenethyl, cyclohexyl, cyclohexylmethyl, 2-pyridylalkyl, or is a side chain of an $\alpha$-amino acid for that peptidase substrate analog,
- X' is H, $C_{1-7}$ alkyl, phenyl, benzyl, phenethyl, cyclohexyl, cyclohexylmethyl, 2-pyridylalkyl or an amino $\beta$ halo $C_{1-6}$ alkylene,

with CHM being an abbreviation for cyclohexylmethylene.

Isosteres of the compounds of formula B include those wherein (a) one or more of the $\alpha$-amino acid residues of the $R_1$ substituent are in their unnatural configuration (when there is a natural configuration) or (b) when the normal peptidic carbamoyl linkage is modified, such as for example, to form $-CH_2NH-$ (reduced),

$$-\overset{\overset{\displaystyle O}{\|}}{C}-N(CH_3)$$

(N-methylamide), $-COCH_2-$ (keto), $-CH(OH)CH_2-$ (hydroxy), $-CH(NH_2)CH_2-$ (amino), $-CH_2CH_2-$ (hydrocarbon). Preferably a compound of the invention should not be in an isosteric form, particularly it is preferred that there be no modified peptidic carbamoyl group in the $R_1$ radical, but if there is, it is preferable to keep the isosteric modifications to a minimum.

A compound of the invention may be in free form, e.g., amphoteric form, or in salt, e.g., acid addition or anionic salt, form. A compound in free form may be converted into a salt form in an art-known manner and vice-versa. Examples of salt forms are the trifluoroacetate, hydrochloride, sodium, potassium and ammonium forms, although the scope of salts embraced herein is not limited thereto, the scope includes all of the salts known to be useful in the art of peptide chemistry.

Unless otherwise stated, the α-amino acid building blocks of these peptidase substrate analogs are preferably in their L-configuration. However, in those instances wherein there is an amide peptide bond between the $CF_2$ and a resulting malonyl moiety, then the α-amino acid building blocks between the $CF_2$ moiety and the malonyl moiety are in their D-configuration.

Before further defining and/or illustrating the scope of the peptidase inhibitors embraced by formula B, it may be convenient to state some of the more basic concepts related to peptides. For example, except for proline, all of the α-amino acids found in proteins have as a common denominator a free carboxyl group and a free unsubstituted amino group on the α-carbon atom (in proline, since proline's α-amino group is substituted it is really an α-imino acid, but for convenience, it will also be spoken of as an α-amino group). Additionally, each α-amino acid has a characteristic "R-group", the R-group being the side chain, or residue, attached to the α-carbon atom of the α-amino acid. For example, the R-group residue for glycine is hydrogen, for alanine it is methyl, for valine it would be isopropyl. (Thus, throughout this specification the $R_2$, $R_3$ moieties are the side chains (or residues) for each indicated α-amino acid or are another radical which is defined for these sites for any given protease inhibitor.) For these specific side chains (or residues) of the involved α-amino acids reference to A.L. Lehninger's text on Biochemistry (particularly Chapter 4) would be helpful.

As a further convenience for defining the scope of the compounds embraced by the generic concept of formula B, as well as the sub-generic concepts relating to each of the individual enzymes involved in this invention, various α-amino acids have been classified into a variety of groups which impart similar functional characteristics for each of the specific enzymes to be inhibited by the peptidase substrates of formula B. These groups are set forth in Table II and the recognized abbreviations for the α-amino acids are set forth in Table I.

TABLE I

| AMINO ACID | SYMBOL |
|---|---|
| Alanine | Ala |
| Arginine | Arg |
| Asparagine | Asn |
| Aspartic acid | Asp |
| Asn + Asp | Asx |
| Cysteine | Cys |
| Glutamine | Gln |
| Glutamic acid | Glu |
| Gln + Glu | Glx |
| Glycine | Gly |
| Histidine | His |
| Isoleucine | Ile |
| Leucine | Leu |
| Lysine | Lys |
| Methionine | Met |
| Phenylalanine | Phe |
| Proline | Pro |
| Serine | Ser |
| Threonine | Thr |
| Tryptophan | Trp |
| Tyrosine | Tyr |
| Valine | Val |
| Norvaline | n-Val |
| Norleucine | n-Leu |
| 1-Naphthylalanine | Nal(1) |
| 2-Indolinecarboxylic acid | Ind |
| Sarcosin | Sar |

TABLE II

Groups:

A: Lys and Arg

B: Glu, Asp

C: Ser, Thr, Gln, Asn, Cys, His, (3-pyrazolyl)Ala, (4-pyrimidinyl)Ala, and N-methyl derivatives

C': Ser, Thr, Gln, Asn and Cys, and their N-methyl derivatives,

D: Pro, Ind

E: Ala, β-Ala, Leu, Ile, Val, n-Val, β-Val, Met, CHM, n-Leu and N-methyl derivatives (β- representing beta)

E': Leu, Ile, n-Val, Met, n-Leu, CHM and their N-methyl derivatives,

F: Phe, Tyr, CHM, O-methyl tyrosine, (3-pyrazolyl)Ala, (4-pyrimidinyl)Ala, Trp, Nal(1), and N-methyl derivatives

F': Phe, Tyr, O-methyl tyrosine, Trp, Nal-(1) and their N-methyl derivatives,

G: Gly, Sar

G': Gly,

J:

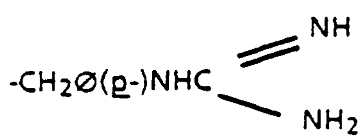
(J-1)

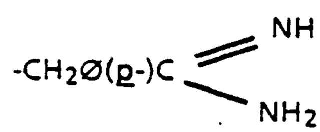
(J-2)

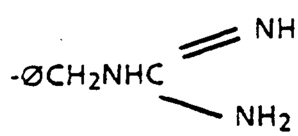
(J-3) and

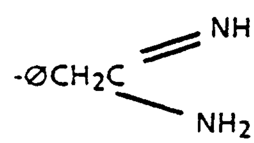
(J-4)

with ⌀, of course, representing phenyl (it being understood that the bond of J1-4 is always attached to an amino acid),

K: Acetyl (Ac), Succinyl (suc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantanesulphonyl ($AdSO_2$), 1-Adamantaneacetyl (AdAc), 2-Carboxybenzoyl (2-CBZ), Phenylacetyl, t-Butylacetyl (Tba), bis[(1-naphthyl)methyl]acetyl (BNMA),

K': is -A-Rz wherein A is $-\overset{O}{\overset{\|}{C}}-$, $-\underset{H}{\underset{|}{N}}-\overset{O}{\overset{\|}{C}}-$, $-O-\overset{O}{\overset{\|}{C}}-$, -or $-\underset{O}{\underset{\|}{\overset{O}{\overset{\|}{S}}}}-$; and Rz is an aryl group containing 6, 10 or 12 carbons suitably substituted by 1 to 3 members selected independently from the group consisting of fluoro, chloro, bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolo, and acylsulfonamido (i.e., acylaminosulfonyl and sulfonylaminocarbonyl) containing from 1 to 15 carbons, provided that when the acylsulfonamido contains an aryl the aryl may be further substituted by a member selected from fluoro, chloro, bromo, iodo and nitro.

In those instances wherein the normal R-group residue of an α-amino acid contains an -OH radical (e.g. serine, threonine and tyrosine), it is to be understood that such radical can be derivatized. For example, in each of the foregoing instances the -OH radical can be converted to an ether. When so-converted, such as for example to their methyl ethers, then such radicals will be referred to as O-methyl serine, O-methyl threonine and O-methyl tyrosine, respectively. These methyl ether-containing side chains may also be depicted as

$$\overset{|}{C}H_2OMe,\ H_3CH\overset{|}{C}-OMe \text{ and } \overset{|}{C}H_2\emptyset-OMe(p),$$

respectively. Similarly, other type derivatives (e.g., N-alkyl derivatives) may also be analogously represented.

In those instances wherein Group K' represents an -A-Rz moiety, it is preferred that A represent -C(=O)- and that Rz represent acylsulfonamido, particularly those wherein the acylsulfonamido contains an aryl moiety (preferably phenyl) substituted by a halogen. The preferred -A-Rz moieties being 4-[(4-chlorophenyl)sulfonylaminocarbonyl]phenylcarbonyl, 4-[(4-bromophenyl)sulfonylaminocarbonyl]-phenylcarbonyl and 4-[phenylsulfonylaminocarbonyl]phenylcarbonyl (said moieties being abbreviated as 4-Cl-ϕ-SAC-Bz, 4-Br-ϕ-SAC-Bz and ϕ-SAC-Bz, respectively). Further, for convenience in defining the scope of the specific protease inhibitors of the sub-generic group Iwb, the term "a ϕ-SAC-Bz" is meant to include 4-Cl-ϕ-SAC-Bz, 4-Br-ϕ-SAC-Bz and ϕ-SAC-Bz.

In those instances wherein the amino-protecting group as defined within Group K is restricted to the $-A-R_z$ moiety, such moiety is designated as K'; K' then being a sub-group of Group K which represents only those moieties defined by $-A-R_z$. In those instances wherein the terminal P-position (as distinguished from the P'-position) nitrogen atom can only bear an amino protecting group selected from Group K', then the definition of $R_1$ will similarly be modified to $R'_1$. Thus, $R'_1$ is defined as an amino protecting group selected from Group K', an α-amino acid or a peptide comprising a number of α-amino acids the terminal nitrogen atom of which bears a member of Group K'. ($R_1$, of course, being as previously defined in that the P-position terminal nitrogen atom may bear any of the Group K or K' protecting groups as well as being an unprotected terminal amine, i.e., its protecting group is optional.)

As used herein the term "alkyl" includes the straight, branched-chain and cyclized manifestations thereof, particularly such moieties as methyl, ethyl, n-butyl, t-butyl, cyclopropyl, n-propyl, pentyl, cyclopentyl, n-hexyl, cyclohexyl and cyclohexylmethyl. The term "aralkyl" includes those aryl moieties attached to a $C_{1-4}$ alkylene, preferably methyl or ethyl. The term "aryl" includes both carbocyclic and heterocyclic moieties. Preferred aralkyl and aryl moieties are phenyl, benzyl, naphthylmethyl, phenethyl, 2-pyridylmethyl.

In the instance wherein the X' moiety of formula B is an amino β-halo $C_{1-6}$ alkylene moiety, the alkylene bears one or two halogens, preferably fluoro, on a carbon atom to which the $NH_2$ group is not directly attached. Illustrative of the preferred radicals are: $-CH_2CH_2CH(CHF_2)NH_2$, $-CH_2CF_2CH_2NH_2$, $-CH_2CH(CHF_2)CH_2NH_2$ and $-CH_2-CHF-CHF-CH_2NH_2$.

In general the peptide of $R_1$ contains 2 to 8 α-amino acids, preferably 2 to 5.

Compounds of this invention which are useful as inhibitors of retroviral proteases required for replication, particularly the HIV-1 and HIV-2 viral proteases, the viruses putatively responsible for causing acquired immuno deficiency syndrome (AIDS) are compounds of the formulae

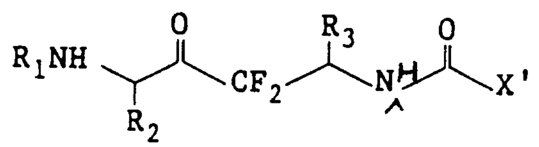

Iwb

and the hydrates, isosteres or the pharmaceutically acceptable salts thereof, wherein

- $R_1$ is H, an amino protecting group, preferably the amino protecting groups are Iva, Boc, CBZ, Tba, 4-Cl-ϕ-SAC-Bz, 4-Br-ϕ-SAC-Bz or ϕ-SAC-Bz,
- $R_2$ is the side chain of the amino acid Phe, Tyr or Leu, or CHM,
- $R_3$ is the side chain of the amino acid Gly and Ile,
- X' is amino-β-halo $C_{1-6}$ alkylene wherein amino-β-halo $C_{1-6}$ alkylene is $-CH_2CH_2CH(CHF_2)NH_2$, $-CH_2CF_2CH_2NH_2$, $-CH_2CH(CHF_2)CH_2NH_2$, and $-CH_2CHFCHFCH_2NH_2$ or is $R_4$ wherein
- $R_4$ is H, $C_{1-6}$ alkyl, phenyl, benzyl, phenethyl, cyclohexyl, cyclohexylmethyl or 2-pyridylmethyl,

The preferred compounds of Formula Iwb are: *CBZ-Phe-[$CF_2$GlyNH]$COCH_2C_6H_5$,
*CBZ-Phe-[$CF_2$GlyNH]$CO(CH_2)_3NH_2$,
*CBZ-Phe-[$CF_2$GlyNH]$COCH_2CF_2CH_2NH_2$,

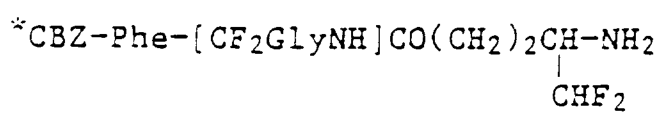

[*In each instance the indicated protecting group (or H) may be replaced by a member of the group consisting of 4-Clϕ-SAC-Bz, 4-Brϕ-SAC-Bz, ϕ-SAC-Bz, Iva, Boc, CBZ and Tba.]

It is to be noted that when X' is amino β-halo-$C_{1-6}$ alkylene, it is preferred that the halo be fluoro, preferably difluoro, and that the fluoro atoms cannot be on the carbon atom to which the $NH_2$ moiety is attached (see last named specific compound). Iva is the moiety $-C(O)CH_2CHMe_2$.

In their end-use application in the treatment of retro-viral infections, the compounds of Formula Iwb will be administered at about 1 to 100 mg per kilogram of body weight per day, preferably administered interperitoneally, intravenously and/or orally.

Having defined the scope of the compounds within the generic invention and within the individual subgeneric groups for each of the individual enzymes, the manner in which such may be prepared will be described and illustrated.

In general, the compounds of formula B may be prepared using standard chemical reactions analogously known in the art. The key intermediates required for the application of standard peptide coupling techniques may be represented by the formula Va or Vb

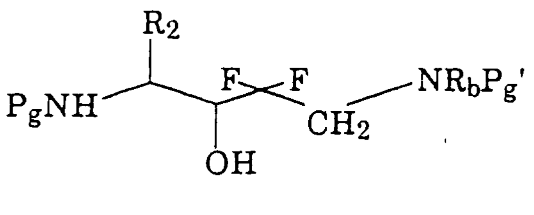

Va

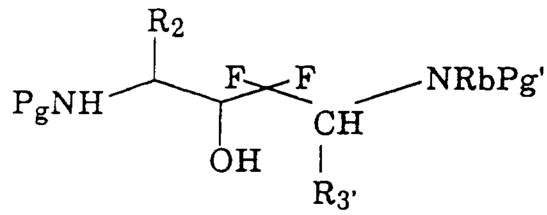

Vb

wherein

- $R'_3$ is as defined for $R_3$ and may be a protected form of the residue of the specific α-amino acid involved,
- $R_2$ is as previously defined, and
- $P_g$ and $P_g'$ are each protecting groups, preferably different from each other so as to facilitate selective removal depending upon the site, nature and sequence of the reactions required to prepare the final compounds from these intermediates; the selection being according to principles well known and understood by those in the art.
- $R_b$ is hydrogen.

In those instances wherein $R'_3$ represents hydrogen the preparation of the required intermediates (Va) is illustrated by Reaction Scheme A. In those instances wherein $R'_3$ is other than hydrogen then the required intermediates (Vb) are prepared by the methods depicted in Reaction Scheme B.

## Reaction Scheme A

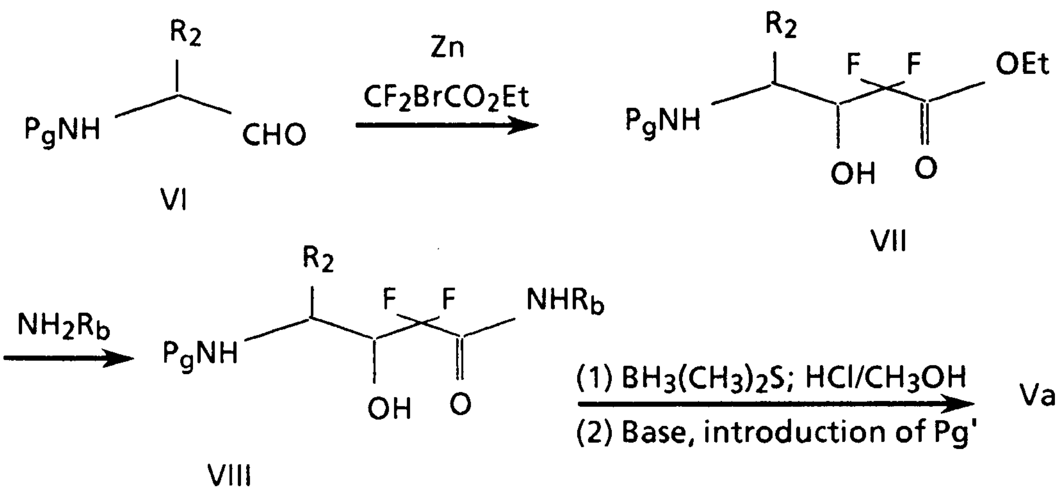


In effecting the steps of Reaction Scheme A it is preferred to start with the aldehyde of formula VI wherein the protecting group is a carbamate preferably wherein $P_g$ isbenzyloxycarbonyl (CBZ). This so-protected aldehyde is subjected to a condensation reaction with an ester of bromodifluoroacetic acid, preferably the ethyl ester in the presence of zinc. Preferably the reaction is conducted in an anhydrous aprotic solvent, e.g., tetrahydrofuran, ether, dimethoxyethane and the like under a nitrogen atmosphere. The reaction mixture is gently heated under reflux conditions, preferably to about 60 °C for about 1-12 hours. The ester (VII) is converted to its primary amide (VIII) by treatment with liquid ammonia or $R_bNH_2$ under anhydrous conditions, preferably using such solvents as anhydrous diethyl ether. The addition of the $R_bNH_2$ or ammonia is initiated at -78 °C and, following completion of the addition, the reaction mixture is slowly

allowed to rise to room temperature. The so-formed amide is chemically reduced to form the free amine. This chemical reduction is easily effected by reacting the amide with a diborane, preferably as a diborane/dimethylsulfide complex, under a nitrogen atmosphere in an anhydrous aprotic solvent (e.g., THF) under reflux conditions. The reduction yields the desired amine, in the form of an acid (e.g., HCl) salt which by pH adjustment yields the free amine which may be suitably protected with an N-protecting group, e.g., $P_g$' is t-butoxy carbonyl using the standard reaction conditions (e.g., $(BOC)_2O$, tetrahydrofuran at room temperature) for protecting the amine. Alternatively the free amine may be subjected to reaction conditions designed to build the desired α-amino acid or peptide moiety on the P' side of the difluoromethylene moiety.

In those instances where $R'_3$ is other than hydrogen then the procedure of Reaction Scheme A is modified to prepare the desired intermediates according to Reaction Scheme B.

Reaction Scheme B

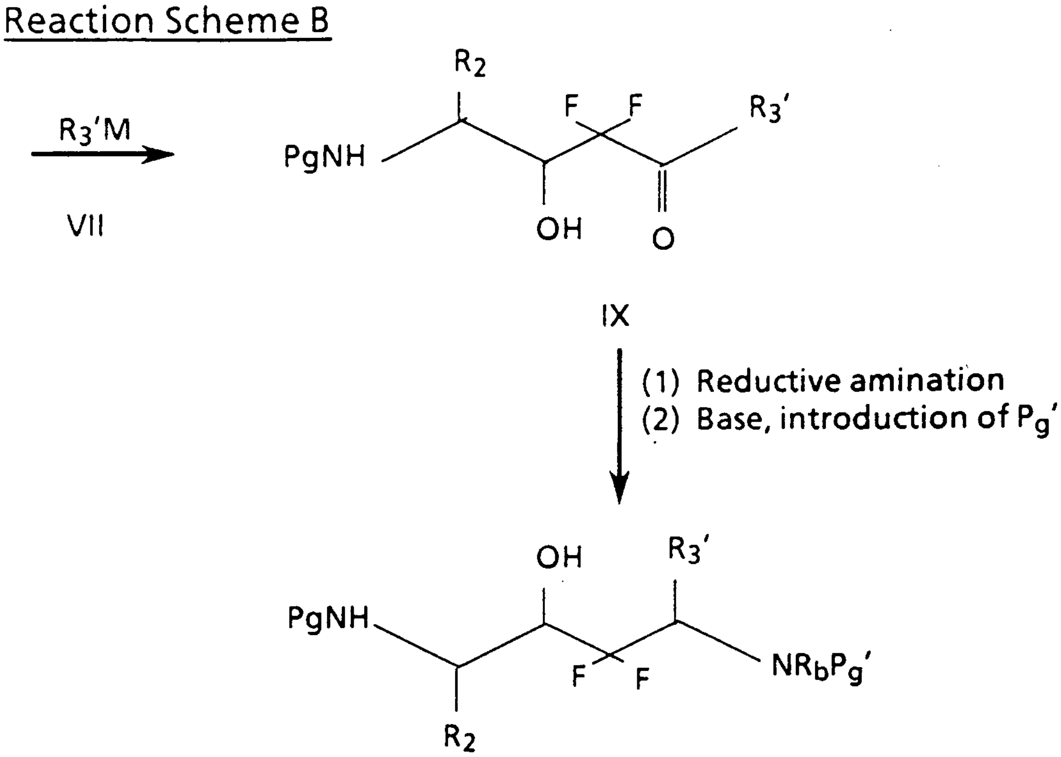


VII

IX

Vb

wherein $R_3$'M is an organometallic reagent, preferably lithium or magnesium coupled to the $R_3$' moiety (other than hydrogen) desired.

The conversion of the ester (VII) to the corresponding $R_3$' bearing ketone with the organometallic reactant is effected by contacting the reactants together under anhydrous conditions at temperatures of about 0°-80°C in an aprotic solvent (e.g., tetrahydrofuran). Upon reaction the temperature is slowly allowed to rise to room temperature and the complex is hydrolysed to yield the desired intermediate ketones (IX) which compounds are subjected to reductive amination procedures well known in the art, such as, for example, the procedure described by Borch (see R.F. Borch, et al., J. Am. Chem. Soc., 93, 2897 (1971). This reductive amination can take place in one or two steps (with isolation of the intermediate imine or enamine). For example, reacting the ketones (IX) with ammonium acetate under slightly acidic conditions in methanol produces the enamine which, when reacted with sodium cyanoborohydride, produces the desired product. Alternatively, the ketones may be treated directly with sodium cyanoborohydride in the presence of ammonium acetate to produce the desired amines (as its HCl salts) which, in either case, may be neutralized and then the $NHR_b$ or $NH_2$ moiety may be protected with an appropriate protecting group.

Having obtained the key intermediates of formula V (a and b) standard α-amino acid or peptide coupling procedures may be conducted to prepare the individual compounds of formula B. In practice it is more convenient to effect coupling on the P' side of the difluoromethylene moiety before coupling the $P_2$-$P_n$ moieties because the CBZ protecting group is generally more stable and this facilitates a less difficult route of synthesis for the desired compounds. In general, this series of reactions may be depicted by Reaction

Scheme C.

Reaction Scheme C

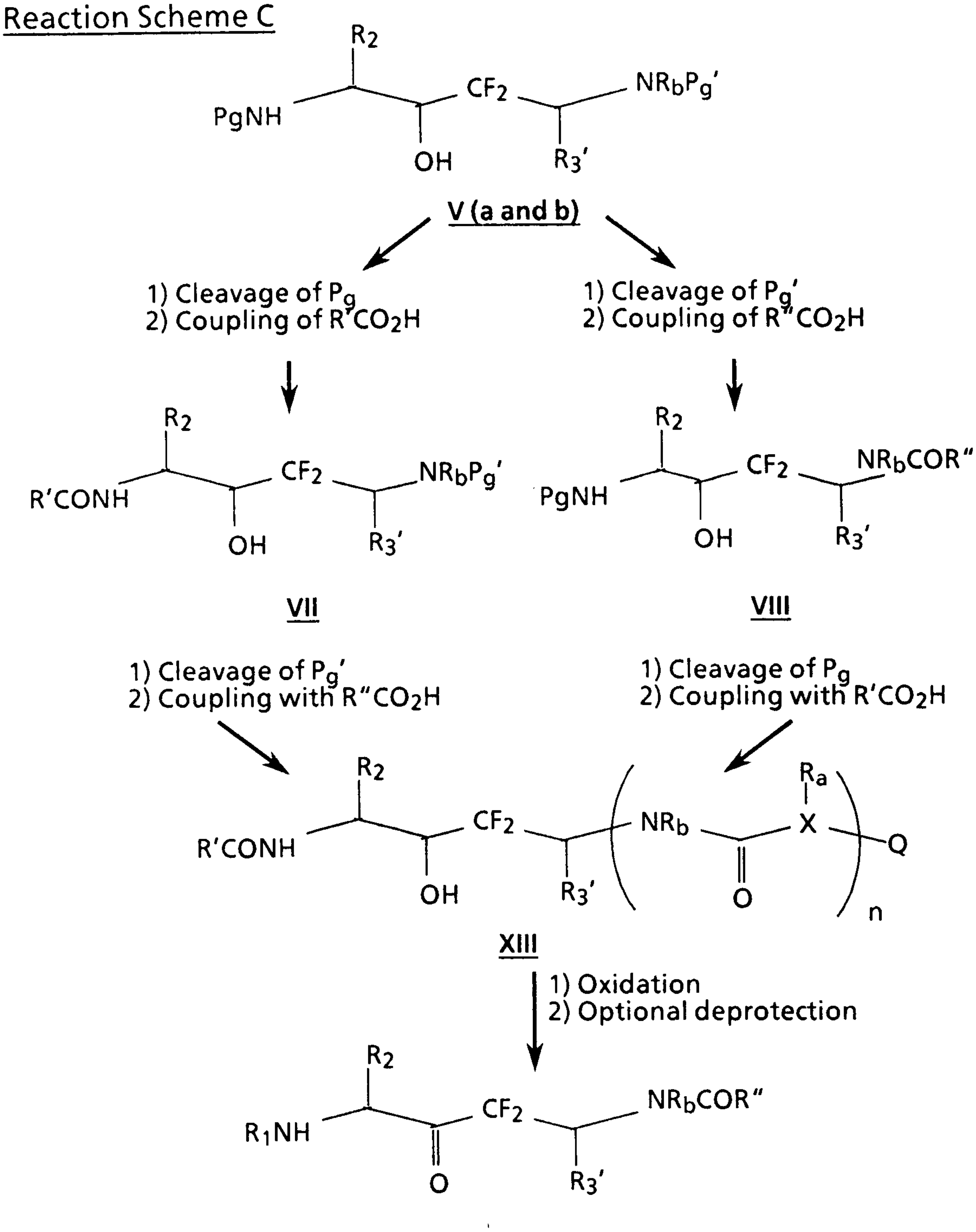

Formula XIV may otherwise be written as

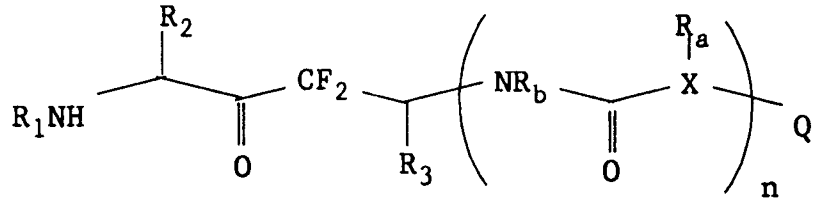

wherein $P_g$, $R_2$, $R_3$, $R_a$, $R_b$, $P_g$' and $R_1$ are as previously defined, $R'CO_2H$ is the equivalent of $R_1OH$ or $R'_1OH$ (except, of course, in the instance wherein $R_1$ is H, coupling on the P-side of the molecule is obviated) and $R''CO_2H$ is the equivalent of

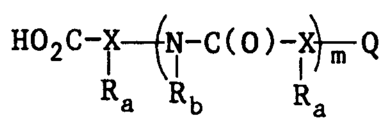

wherein m is n minus one, i.e., R" is

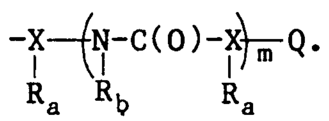

The oxidation may be effected via the well-known Swern oxidation procedure, or with a modified Jones reaction using pyridinium dichromate, or a chromic anhydride-pyridinium complex, or with 1,1,1-triacetoxy-2,1-benzoxiodol. Of course, if there are any protecting groups on the residues of the α-amino acid building blocks, such protecting groups may be removed after oxidation. The coupling procedures are effected according to standard procedures well known in the art.

In general the Swern oxidation is effected by reacting about 2 to 10 equivalents of dimethylsulfoxide (DMSO) with about 1 to 6 equivalents of trifluoromethylacetic anhydride [$(CF_3CO)_2O$] or oxalyl chloride [-$(COCl)_2$], said reactants being dissolved in an inert solvent, e.g., methylene chloride ($CH_2Cl_2$), said reactor being under an inert atmosphere (e.g., nitrogen or equivalently functioning gas) under anhydrous conditions at temperatures of about -80 °C to -50 °C to form an *in situ* sulfonium adduct to which is added about 1 equivalent of the appropriate alcohols, i.e., from compounds VII and VIII by the coupling with $R''CO_2H$ and $R'CO_2H$, respectively, having the formula

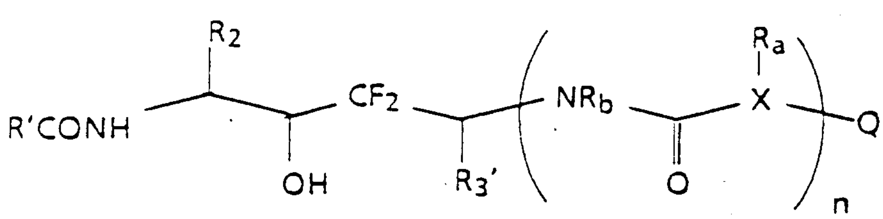

XIII

Preferably, the alcohols are dissolved in an inert solvent, e.g., $CH_2Cl_2$ or minimum amounts of DMSO, and the reaction mixture is allowed to warm to about -50 °C (for about 10-20 minutes) and then the reaction is completed by adding about 3 to 10 equivalents of a tertiary amine, e.g., triethylamine, N-methyl morpholine, etc.

In general, the modified Jones oxidation procedure may conveniently be effected by reacting the alcohols XIII with pyridinium dichromate by contacting the reactants together in a water-trapping molecular sieve powder (e.g., a grounded 3 Angström molecular sieve), wherein said contact is in the presence of glacial acetic acid at about 0 °C to 50 °C, preferably at room temperature followed by isolation and then

optionally removing amine protecting groups.

Alternatively, 1 to 5 equivalents of a chronic anhydride-pyridine complex (i.e., a Sarett reagent prepared *in situ* (see Fieser and Fieser "Reagents for Organic Synthesis" Vol. 1, pp. 145 and Sarett, et al., J.A.C.S. 25, 422, (1953)) said complex being prepared *in situ* in an inert solvent (e.g., $CH_2Cl_2$) in an inert atmosphere under anhydrous conditions at 0°C to 50°C to which complex is added 1 equivalent of the alcohols (XII) allowing the reactants to interact for about 1 to 15 hours, followed by isolation and optionally removing amine protecting groups.

Another alternative process for converting the alcohols (XII) to the desired ketones (XIV) is an oxidation reaction which employs periodane (i.e., 1,1,1-triacetoxy-2,1-benzoxiodol, (see Dess Martin, J. Org. Chem., 48, 4155, (1983)). This oxidation is effected by contacting about 1 equivalent of the alcohols (XII) with 1 to 5 equivalents of periodane (preferably 1.5 equivalents), said reagent being in suspension in an inert solvent (e.g., methylene chloride) under an inert atmosphere (preferably nitrogen) under anhydrous conditions at 0°C to 50°C (preferably room temperature) and allowing the reactants to interact for about 1 to 48 hours. Optional deprotection of the amine protecting groups may be effected as desired after the ketones have been isolated.

The preparation of the compounds of Formula Iwb follows the same general chemical pathways as outlined and described in Reaction Schemes A and B for the preparation of the key intermediates Va and Vb and also, with minor modifications, essentially follows the reaction pathways of Reaction Scheme C for those compounds wherein solid-phase chemistry is not required (i.e., for those compounds of Formula Iwb) and for those compounds which require solid-phase chemistry for the attachment of the $R_1$ moieties, the required intermediates (XIX) are prepared essentially along the same lines, with slight modifications, as outlined in Reaction Scheme C in preparing compounds XIII. The preparation of the Iwb compounds is more specifically illustrated in Reaction Scheme D

Reaction Scheme D

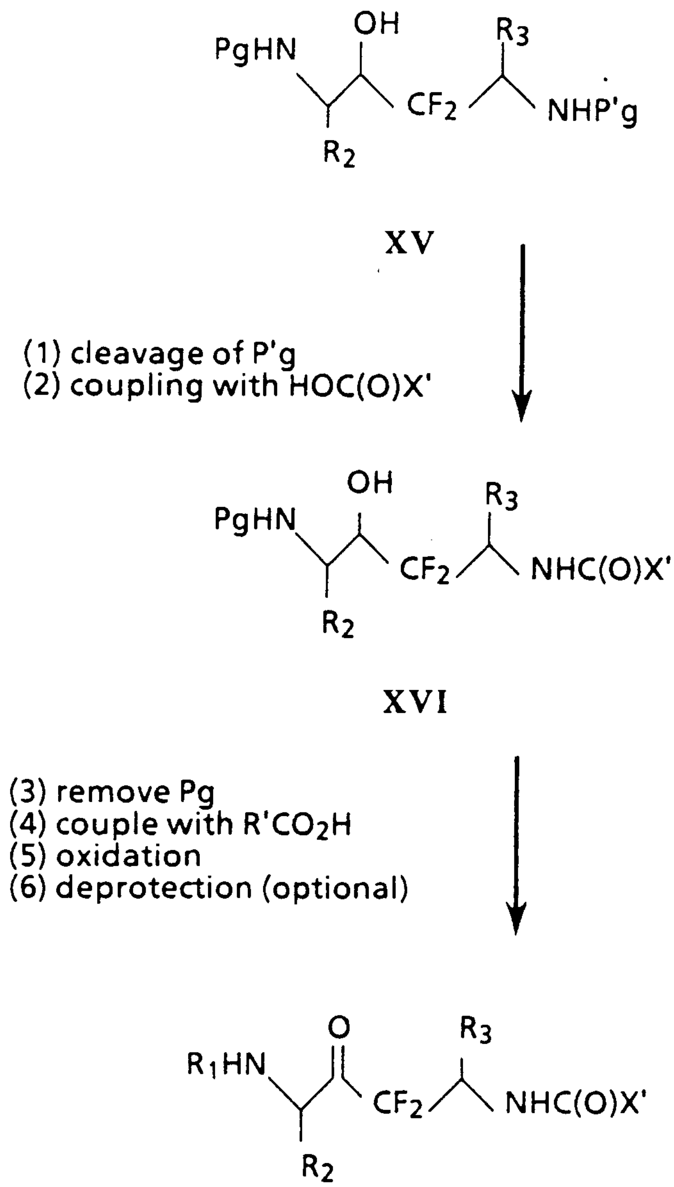


XV

XVI

XVII

wherein $R_1$, $R_2$, $R_3$, R'COOH and X' are as previously defined.

The solid phase sequential procedure can be performed using established automated methods such as by use of an automated peptide synthesizer. In this procedure an amino protected amino acid is bound to a resin support at the carboxy terminal end, the amino acid is deprotected at the amino position at which a peptide linkage is desired, the amino group neutralized with a base and the next amino protected amino acid in the desired sequence is coupled in a peptide linkage. The deprotection, neutralization and coupling steps are repeated until the desired polypeptide is synthesized. The compounds of the present invention are thus synthesized from their carboxy terminal end to their amino terminal end. The amino protected amino acid can be a conventional amino acid, a derivative or isomer thereof, or a spacer group. The resin support employed can be any suitable resin conventionally employed in the art for the solid phase preparation of polypeptides. The preferred resin is polystyrene which has been cross-linked with from about 0.5 to about 3% divinyl benzene, which has been either benzhydryl-amidated, chloromethylated or hydroxymethylated to provide sites for amide or ester formation with the initially introduced amino protected amino acid.

An example of a hydroxymethyl resin is described by Bodansky et al. [Chem. Ind. (London) 38, 1597-98 (1966)]. The preparation of chloromethyl and benzhydrylamine resins are described by Stewart et al. ["Solid Phase Peptide Synthesis", 2nd Edition, Pierce Chemical Co., Rockford, Illinois (1984), Chapter 2, pp. 54-55]. Many of these resins are available commercially. In general, the amino protected amino acid which is desired on the carboxy-terminal end of the peptide is bound to the resin using standard procedures and practices as are well known and appreciated in the art. For example, the amino protected amino acid can be bound to the resin by the procedure of Gisin [Helv. Chem. Acta, 56, 1476 (1973)]. When it is desired to use a resin containing a benzhydrylamine moiety as the resin binding site an amino protected amino acid is coupled to the resin through an amide linkage between its α-carboxylic acid and the amino moiety of the resin. This coupling is effected using standard coupling procedures as described below. Many resin-bound amino acids are available commercially.

The α-amino protecting group employed with each amino acid introduced into the polypeptide sequence may be any such protecting group known in the art. Among the classes of amino protecting groups contemplated are: (1) acyl type protecting groups such as formyl, trifluoroacetyl, phthalyl, p-toluenesulfonyl (tosyl), benzenesulfonyl, nitrophenylsulfenyl, tritylsulfenyl, o-nitrophenoxyacetyl, and α-chlorobutyryl; (2) aromatic urethane type protecting groups such as benzyloxycarbonyl and substituted benzyloxycarbonyls such as p-chlorobenzyloxycarbonyl, p-methoxybenzyloxycarbonyl, p-nitrobenzyloxycarbonyl, p-bromobenzyloxycarbonyl, 1-(p-biphenylyl)-1-methylethoxycarbonyl, α-, α-dimethyl-3,5-dimethoxybenzyloxycarbonyl, and benzhydryloxycarbonyl; (3) aliphatic urethane protecting groups such as tert-butyloxycarbonyl (Boc), diisopropylmethoxycarbonyl, isopropyloxycarbonyl, ethoxycarbonyl, and allyloxycarbonyl; (4) cycloalkyl urethane type protecting groups such as cyclopentyloxycarbonyl, adamantyloxycarbonyl, and cyclohexyloxycarbonyl; (5) thio urethane type protecting groups such as phenylthiocarbonyl; (6) alkyl type protecting groups such as triphenylmethyl (trityl) and benzyl (Bzl); (7) trialkylsilane protecting groups such as trimethylsilane. The preferred α-amino protecting group is tert-butyloxycarbonyl (Boc). The use of Boc as an α-amino protecting group for amino acids is described by Bodansky et al. in "The Practice of Peptide Synthesis", Springer-Verlag, Berlin (1984), p. 20.

Following the coupling of the amino protected amino acid to the resin support, the α-amino protecting group is removed using any suitable procedure such as by using trifluoroacetic acid, trifluoroacetic acid in dichloromethane, or HCl in dioxane. The deprotection is carried out at a temperature of between 0 °C and room temperature. Other standard cleaving reagents may be used for removal of specific amino protecting groups under conditions well known and appreciated in the art.

After removal and neutralization of the α-amino protecting group the next desired amino-protected amino acid is coupled through a peptide linkage. This deprotection, neutralization and coupling procedure is repeated until a polypeptide of the desired sequence is obtained. Alternatively, multiple amino acid groups may be coupled by the solution method prior to coupling with the resin supported amino acid sequence. The selection and use of an appropriate coupling reagent is within the skill of the ordinary practitioner in the art. Particularly suitable coupling reagents where the amino acid to be added is Gln, Asn, or Arg are N,N-dicyclohexylcarbodiimide and 1-hydroxybenzotriazole. The use of these reagents prevents nitrile and lactam formation. Other coupling agents are (1) carbodiimides (e.g., N,N-dicyclohexylcarbodiimide and N-ethyl-N'-(γ-dimethylaminopropylcarbodiimide); (3) ketenimines; (4) isoxazolium salts (e.g., N-ethyl-5-phenylisoxazolium-3-sulfonate); (5) monocyclic nitrogen containing heterocyclic amides of aromatic character containing one through four nitrogens in the ring such as imidazolides, pyrazolides, and 1,2,4-triazolides (specific heterocyclic amides that are useful include N,N-carbonyldiimidazole and N,N-carbonyl-di-1,2,4-triazole);(6) alkoxylated acetylene (e.g., ethoxyacetylene); (7) reagents which form a mixed anhydride with the carboxyl moiety of the amino acid (e.g., ethylchloroformate and isobutylchloroformate) or the the symmetrical anhydride of the amino acid to be coupled (e.g., Boc-Ala-O-Ala-Boc); (8) nitrogen containing heterocyclic compounds having a hydroxy group on one ring nitrogen (e.g., N-hydroxyphthalimide, N-hydroxysuccinimide, and 1-hydroxybenzotriazole). Other activating reagents and their use in peptide coupling are described by Kapoor [J. Pharm. Sci., 59, 1-27 (1970)]. The generally preferred coupling method for the amino acids used in the present invention is the use of the symmetrical anhydride as the coupling agent.

The preferred coupling method for Gln, Asn and Arg is to react the protected amino acid, or derivatives or isomers thereof, with N,N-dicyclohexylcarbodiimide and 1-hydroxybenzotriazole (1:1) in N,N-dimethylformamide (DMF) in the presence of the resin or resin-bound amino acid or peptide. The preferred coupling method for other amino acids involves reacting the protected amino acid, or derivative or isomer thereof, with N,N-dicyclohexylcarbodiimide in dichloromethane to form the symmetrical anhydride. The symmetrical anhydride in then introduced into the solid phase reactor containing the resin or resin-bound amino acid or peptide, and the coupling is carried out in a medium of DMF, or dichloromethane, or DMF: dichloromethane (1:1). A medium of DMF is preferred. The success of the coupling reaction at each stage of the synthesis is

monitored by a ninhydrin test as described by Kaiser et al. [Analyt. Biochem. 34, 595 (1970)]. In cases where incomplete coupling occurs, the coupling procedure is repeated. If the coupling is still incomplete, the deprotected amine is capped with a suitable capping reagent to prevent its continued synthesis. Suitable capping reagents and the use thereof are well known and appreciated in the art. Examples of suitable capping reagents are acetic anhydride and acetylimidazole as described by Stewart et al. ["Solid Phase Peptide Synthesis", 2nd Ed., Pierce Chemical Co., Rockford, Ill. (1984), Chapter 2, p. 73].

After the desired amino acid sequence has been obtained, the peptide is cleaved from the resin. This can be effected by procedures which are well known and appreciated in the art, such as by hydrolysis of the ester or amide linkage to the resin. It is preferred to cleave the peptide from the benzhydrylamine resin with a solution of dimethyl sulfide, p-cresol, thiocresol, or anisole in anhydrous hydrogen fluoride. The cleavage reaction is preferably carried out at temperatures between about 0°C and about room temperature, and is allowed to continue preferably from between about 5 minutes to about 5 hours.

As is known in the art of solid phase peptide synthesis, many of the amino acids bear side chain functionalities requiring protection during the preparation of the peptide. The selection and use of an appropriate protecting group for these side chain functionalities is within the ability of those skilled in the art and will depend upon the amino acid to be protected and the presence of other protected amino acid residues in the peptide. The selection of such a side chain protecting group is critical in that it must not be removed during the deprotection and coupling steps of the synthesis. For example, when Boc is used as the $\alpha$-amino protecting group, the following side chain protecting groups are suitable: p-toluenesulfonyl (tosyl) moieties can be used to protect the amino side chains of amino acids such as Lys and Arg; p-methylbenzyl, acetamidomethyl, benzyl (Bzl), or t-butylsulfonyl moieties can be used to protect the sulfide containing side chains of amino acids such as cysteine, homocysteine, penicillamine and the like or derivatives thereof; benzyl (Bzl) or cyclohexyl ester moieties can be used to protect carboxylic acid side chains of amino acids such as Asp, Glu; a benzyl (Bzl) ether can be used to protect the hydroxy containing side chains of amino acids such as Ser and Thr; and a 2-bromocarbobenzoxy (2Br-Z) moiety can be used to protect the hydroxy containing side chains of amino acids such as Tyr. These side chain protecting groups are added and removed according to standard practices and procedures well known in the art. It is preferred to deprotect these side chain protecting groups with a solution of anisole in anhydrous hydrogen fluoride (1:10). Typically, deprotection of side chain protecting groups is performed after the peptide chain synthesis is complete but these groups can alternatively be removed at any other appropriate time. It is preferred to deprotect these side chains at the same time as the peptide is cleaved from the resin.

The compounds are then isolated and purified by standard techniques. The desired amino acids, derivatives and isomers thereof can be obtained commercially or can be synthesized according to standard practices and procedures well known in the art.

The foregoing describes in detail the generic and specific aspects of the scope of the invention as well as the manner of making and using the invention.

By following the techniques referenced above, as well as by utilization of other known techniques, as well as by comparison with compounds known to be useful for treatment of the above-mentioned disease states, it is believed that adequate material is available to enable one of ordinary skill in the art to practice the invention. Of course, in the end-use application of the compounds of this invention, the compounds are preferably formulated into suitable pharmaceutical preparations such as tablets, capsules or elixirs, for oral administration or in sterile solutions or suspensions for parenteral administration. The compounds of this invention can be administered to patients (animals and human) in need of such treatment in a dosage range of 5 to 500 mg per patient generally given several times, thus giving a total daily dose of from 5 to 2000 mg per day. As stated above, the dose will vary depending on severity of disease, weight of patient and other factors which a person skilled in the art will recognize.

Typically the compounds described above are formulated into pharmaceutical compositions as discussed below.

About 10 to 500 mg of a compound or mixture of compounds of formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it

may contain in addition to materials of the above type, a liquid carrier such as fatty oil. Various other materials may he present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

**Claims**

**Claims for the following Contracting States : AT, BE, CH, LI, DE, FR, GB, GR, IT, LU, NL, SE**

**1.** A compound of the formula

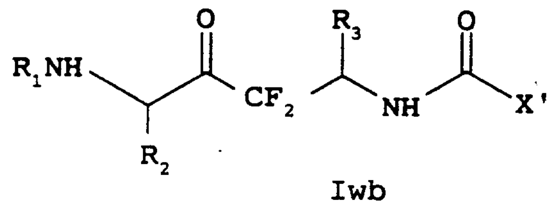

Iwb

and the hydrates, isosteres or the pharmaceutically acceptable salts thereof, wherein

- $R_1$ is H, or is an amino protecting group selected from :
  - Isovaleryl (Iva) ;
  - t-Butyloxycarbonyl (Boc) ;
  - Carbobenzoxy (CbZ) ;
  - t-Butylacetyl (Tba) ;
  - 4-[4-chlorophenyl )sulfonylaminocarbonyl]phenylcarbonyl (4-Cl-Φ-SAC-Bz) ;
  - 4-[4-bromophenyl )sulfonylaminocarbonyl]phenylcarbonyl (4-Br-Φ-SAC-Bz) or
  - 4-[phenylsulfonylaminocarbonyl]phenylcarbonyl (Φ-SAC-Bz) ;
- $R_2$ is the side chain of the amino acids Phe, Tyr or Leu or is cyclohexylmethyl ;
- $R_3$ is the side chain of the amino acids Gly or Ile ;
- X' is amino-$\beta$-halo $C_{1-6}$ alkylene wherein amino-$\beta$-halo $C_{1-6}$ alkylene is $-CH_2CH_2CH(CHF_2)-NH_2$, $-CH_2CF_2CH_2NH_2$, $-CH_2CH(CHF_2)CH_2NH_2$, $-CH_2CHFCHFCH_2NH_2$, or is $R_4$ wherein $R_4$ is H, $C_{1-6}$ alkyl, phenyl, benzyl, phenethyl, cyclohexyl, cyclohexylmethyl or 2-pyridylmethyl.

**2.** A compound of claim 1, said compound being

Pg-Phe-[$CF_2$GlyNH]$COCH_2C_6H_5$,

Pg-Phe-[$CF_2$GlyNH]$CO(CH_2)_3NH_2$,

Pg-Phe-[$CF_2$GlyNH]$COCH_2CF_2CH_2NH_2$, or

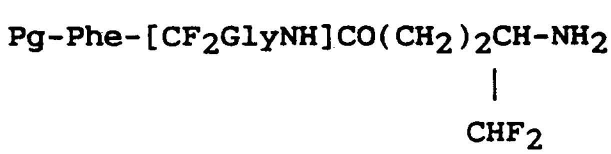

wherein Pg is
4-[4-chlorophenyl)sulfonylaminocarbonyl]phenylcarbonyl (4-Cl-Φ-SAC-Bz) ;
4-[4-bromophenyl )sulfonylaminocarbonyl]phenylcarbonyl (4-Br-Φ-SAC-Bz) or
4-[phenylsulfonylaminocarbonyl]phenylcarbonyl (Φ-SAC-Bz).

**3.** A use of a compound of formula Iwb as defined in claim 1 or 2 for the preparation of a pharmaceutical preparation for treating acquired immuno deficiency syndrome.

**4.** A compound according to claim 1 or 2 or a mixture thereof for use as a pharmaceutically active compound.

**Claims for the following Contracting State : ES**

**1.** Process for preparing the compounds of formula

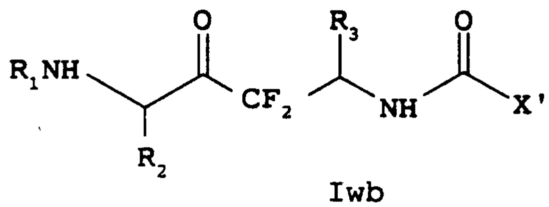

Iwb

and the hydrates, isosteres or the pharmaceutically acceptable salts thereof, wherein

$R_1$ is H, or is an amino protecting group selected from :
Isovaleryl (Iva) ;
t-Butyloxycarbonyl (Boc) ;
Carbobenzoxy (CbZ) ;
t-Butylacetyl (Tba) ;
4-[4-chlorophenyl)sulfonylaminocarbonyl]phenylcarbonyl (4-Cl-Φ-SAC-Bz) ;
4-[4-bromophenyl)sulfonylaminocarbonyl]phenylcarbonyl (4-Br-Φ-SAC-Bz) or
4-[phenylsulfonylaminocarbonyl]phenylcarbonyl (Φ-SAC-Bz) ;

$R_2$ is the side chain of the amino acids Phe, Tyr or Leu or is cyclohexylmethyl ;

$R_3$ is the side chain of the amino acids Gly or Ile ;

X' is amino-$\beta$-halo $C_{1-6}$ alkylene wherein amino-$\beta$-halo $C_{1-6}$ alkylene is $-CH_2CH_2CH(CHF_2)-NH_2$, $-CH_2CF_2CH_2NH_2$, $-CH_2CH(CHF_2)CH_2NH_2$, $-CH_2CHFCHFCH_2NH_2$, or is $R_4$ wherein $R_4$ is H, $C_{1-6}$ alkyl, phenyl, benzyl, phenethyl, cyclohexyl, cyclohexylmethyl or 2-pyridylmethyl,

which comprises

1) cleaving Pg' from the compound of formula XV

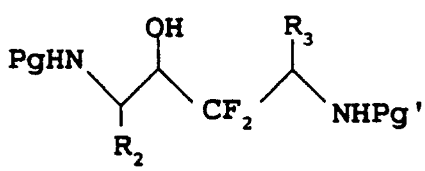

XV

in which $R_2$ and $R_3$ are as hereinabove defined and Pg and Pg' are each protecting groups ;

2) coupling the resulting compound with HOC(O)X', in which X' is as hereinabove defined to yield a compound of formula XVI

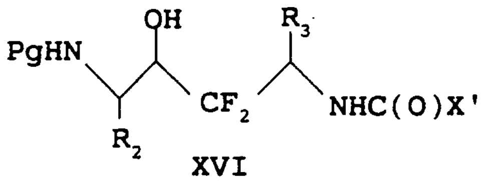

XVI

3) optionally removing Pg from this compound ;
4) coupling it with $R'CO_2H$, $R'CO_2H$ being the equivalent of $R_1OH$ ;
5) oxidizing the resulting compound ;
6) optionally deprotecting the resulting compound to yield the compound of formula Iwb.

**2.** Process according to claim 1, in which a compound of formula XV in which :

- the moiety

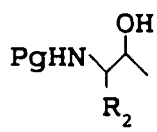

represents
Pg-Phe- wherein Pg is
4-[4-chlorophenyl)sulfonylaminocarbonyl]phenyl carbonyl(4-Cl-Φ-SAC-Bz) ;
4-[4-bromophenyl)sulfonylaminocarbonyl]phenylcarbonyl (4-Br-Φ-SAC-Bz) or
4-[phenylsulfonylaminocarbonyl]phenylcarbonyl (Φ-SAC-Bz) ;
- $R_3$ is the side chain of Gly ;
- Pg' is as defined in claim 1, is reacted with a compound of formula : HOC(O)X' in which X' represents a group selected from
- $CH_2C_6H_5$,
- $(CH_2)_3NH_2$,
- $(CH_2)COCH_2CF_2CH_2NH_2$, or

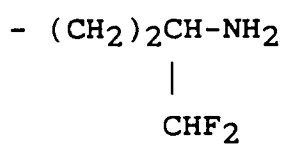

for yielding the following compounds :
Pg-Phe-[$CF_2$GlyNH]$COCH_2C_6H_5$,
Pg-Phe-[$CF_2$GlyNH]$CO(CH_2)_3NH_2$,
Pg-Phe-[$CF_2$GlyNH]$COCH_2CF_2CH_2NH_2$, or

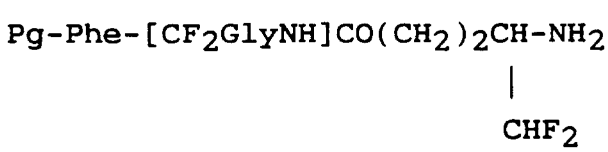

in which Pg is as hereinabove defined.

**3.** A use of a compound of formula Iwb as defined in claim 1 or 2 for the preparation of a pharmaceutical preparation for treating acquired immuno deficiency syndrome.

**Patentansprüche**

**Patentansprüche für folgende Vertragsstaaten : AT, BE, CH, LI, DE, FR, GB, GR, IT, LU, NL, SE**

1. Verbindung der Formel

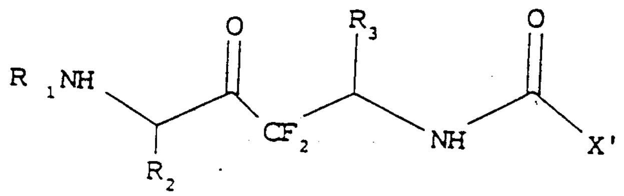

Iwb

und ihre Hydrate, Isostere oder pharmazeutisch verträglichen Salze, wobei

$R_1$ ein Wasserstoffatom oder eine Aminoschutzgruppe bedeutet, ausgewählt aus einer:
Isovalerylgruppe (Iva),
t-Butyloxycarbonylgruppe (Boc),
Carbobenzoxygruppe (Cbz),
t-Butylacetylgruppe (Tba),
4-[(4-Chlorphenyl)sulfonylaminocarbonyl]phenylcarbonylgruppe (4-ClΦ-SAC-Bz),
4-[(4-Bromphenyl)sulfonylaminocarbonyl]phenylcarbonylgruppe (4-BrΦ-SAC-Bz), oder
4-[Phenylsulfonylaminocarbonyl]phenylcarbonylgruppe (Φ-SAC-Bz);

$R_2$ die Seitenkette der Aminosäuren Phe, Tyr oder Leu bedeutet oder eine Cyclohexylmethylgruppe darstellt,

$R_3$ die Seitenkette der Aminosäuren Gly oder Ile ist;

X' ein Amino-$\beta$-halogen-$C_1$-$C_6$-Alkylenrest ist, wobei der Amino-$\beta$-halogen-$C_1$-$C_6$-Alkylenrest $-CH_2CH_2CH(CHF_2)NH_2$, $-CH_2CF_2CH_2NH_2$, $-CH_2CH(CHF_2)CH_2NH_2$, $-CH_2CHFCHFCH_2NH_2$ ist, oder $R_4$ ist, wobei $R_4$ ein Wasserstoffatom, einen $C_1$-$C_6$-Alkylrest, eine Phenylgruppe, eine Benzylgruppe, eine Phenethylgruppe, eine Cyclohexylgruppe, eine Cyclohexylmethylgruppe oder eine 2-Pyridylmethylgruppe bedeutet.

2. Verbindung nach Anspruch 1, nämlich
Pg-Phe-[$CF_2$GlyNH]$COCH_2C_6H_5$,
Pg-Phe-[$CF_2$GlyNH]$CO(CH_2)_3NH_2$,
Pg-Phe-[$CF_2$GlyNH]$COCH_2CF_2CH_2NH_2$, oder

Pg-Phe-[$CF_2$GlyNH]$CO(CH_2)_2CH$-$NH_2$,
|
$CHF_2$

wobei Pg eine
4-[(4-Chlorphenyl)sulfonylaminocarbonyl]phenylcarbonylgruppe (4-ClΦ-SAC-Bz),
4-[(4-Bromphenyl)sulfonylaminocarbonyl]phenylcarbonylgruppe (4-BrΦ-SAC-Bz), oder
4-[Phenylsulfonylaminocarbonyl]phenylcarbonylgruppe (Φ-SAC-Bz) ist.

3. Verwendung einer Verbindung der Formel Iwb nach Anspruch 1 oder 2 für die Herstellung eines Arzneimittels zur Behandlung des erworbenen Immunschwächesyndroms.

4. Verbindung nach Anspruch 1 oder 2 oder ein Gemisch davon zur Verwendung als eine pharmazeutisch wirksame Verbindung.

**Patentansprüche für folgenden Vertragsstaat : ES**

**1.** Verfahren zur Herstellung der Verbindungen der Formel

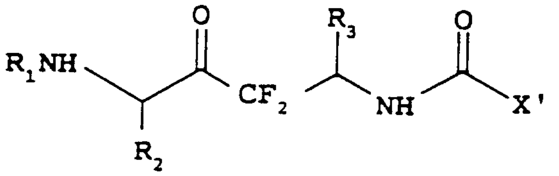

Iwb

und ihrer Hydrate, Isostere oder pharmazeutisch verträglichen Salze, wobei

$R_1$ ein Wasserstoffatom oder eine Aminoschutzgruppe bedeutet, ausgewählt aus einer: Isovalerylgruppe (Iva), t-Butyloxycarbonylgruppe (Boc), Carbobenzoxygruppe (Cbz), t-Butylacetylgruppe (Tba), 4-[(4-Chlorphenyl)sulfonylaminocarbonyl]phenylcarbonylgruppe (4-ClΦ-SAC-Bz), 4-[(4-Bromphenyl)sulfonylaminocarbonyl]phenylcarbonylgruppe (4-BrΦ-SAC-Bz), oder 4-[Phenylsulfonylaminocarbonyl]phenylcarbonylgruppe (Φ-SAC-Bz);

$R_2$ die Seitenkette der Aminosäuren Phe, Tyr oder Leu bedeutet oder eine Cyclohexylmethylgruppe darstellt,

$R_3$ die Seitenkette der Aminosäuren Gly oder Ile ist;

X' ein Amino-$\beta$-halogen-$C_1$-$C_6$-Alkylenrest ist, wobei der Amino-$\beta$-halogen-$C_1$-$C_6$-Alkylenrest $-CH_2CH_2CH(CHF_2)NH_2$, $-CH_2CF_2CH_2NH_2$, $-CH_2CH(CHF_2)CH_2NH_2$, $-CH_2CHFCHFCH_2NH_2$ ist, oder $R_4$ ist, wobei $R_4$ ein Wasserstoffatom, einen $C_1$-$C_6$-Alkylrest, eine Phenylgruppe, eine Benzylgruppe, eine Phenethylgruppe, eine Cyclohexylgruppe, eine Cyclohexylmethylgruppe oder eine 2-Pyridylmethylgruppe bedeutet,

umfassend

1) Abspaltung von Pg' von der Verbindung der Formel XV

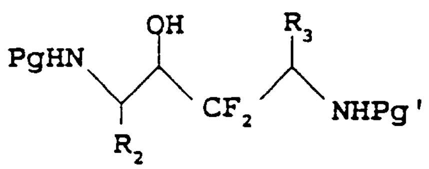

(XV)

in der $R_2$ und $R_3$ wie vorstehend definiert sind und Pg und Pg' jeweils Schutzgruppen sind;

2) Kupplung der erhaltenen Verbindung mit HOC(O)X', in der X' die vorstehend angegebene Bedeutung hat, wobei man eine Verbindung der Formel (XVI) erhält

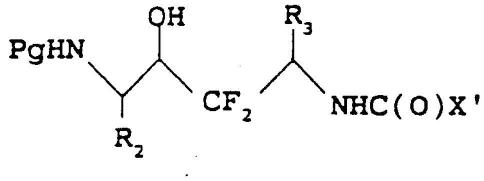

(XVI)

3) gegebenenfalls Entfernung von Pg von dieser Verbindung;

4) Kupplung mit $R'CO_2H$, wobei $R'CO_2H$ äquivalent mit $R_1OH$ ist;

5) Oxidation der erhaltenen Verbindung;

6) gegebenenfalls Entfernung der Schutzgruppen von der erhaltenen Verbindung, wobei man die Verbindung der Formel Iwb erhält.

**2.** Verfahren nach Anspruch 1, wobei eine Verbindung der Formel XV, in der:

- die Einheit

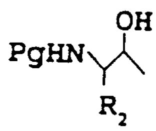

Pg-Phe- darstellt, wobei Pg eine 4-[(4-Chlorphenyl)sulfonylaminocarbonyl]phenylcarbonylgruppe (4-ClΦ-SAC-Bz), 4-[(4-Bromphenyl)sulfonylaminocarbonyl]phenylcarbonylgruppe (4-BrΦ-SAC-Bz), oder 4-[Phenylsulfonylaminocarbonyl]phenylcarbonylgruppe (Φ-SAC-Bz) ist;
- $R_3$ die Seitenkette von Gly ist;
- Pg' wie in Anspruch 1 definiert ist,

mit einer Verbindung der Formel HOC(O)X' umgesetzt wird, in der X' die Gruppen
- $CH_2C_6H_5$,
- $(CH_2)_3NH_2$,
- $(CH_2)COCH_2CF_2CH_2NH_2$, oder

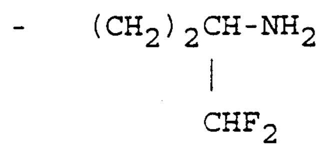

darstellt,
wobei man die folgenden Verbindungen erhält
Pg-Phe-[$CF_2$GlyNH]$COCH_2C_6H_5$,
Pg-Phe-[$CF_2$GlyNH]$CO(CH_2)_3NH_2$,
Pg-Phe-[$CF_2$GlyNH]$COCH_2CF_2CH_2NH_2$, oder

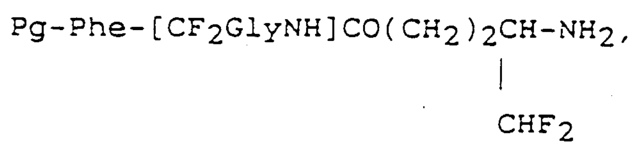

in denen Pg die Vorstehend angegebene Bedeutung hat.

**3.** Verwendung einer Verbindung der Formel Iwb gemäß der Definition in Anspruch 1 oder 2 für die Herstellung eines Arzneimittels zur Behandlung des erworbenen Immunschwächesyndroms.

**Revendications**
**Revendications pour les Etats contractants suivants : AT, BE, CH, LI, DE, FR, GB, GR, IT, LU, NL, SE**

**1.** Composé de formule

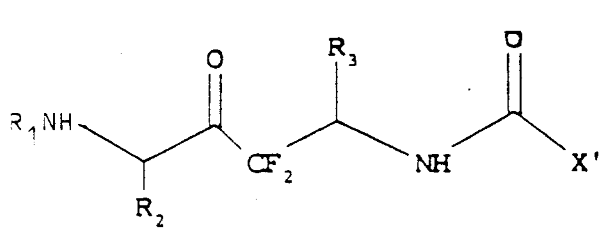

**Iwb**

et ses hydrates, isostères ou sels pharmaceutiquement acceptables, où

- $R_1$ est H, ou un groupe amino-protecteur choisi parmi :
  - isovaléryle (Iva) ;
  - t-butyloxycarbonyle (Boc) ;
  - carbobenzoxy (Cbz) ;
  - t-butylacétyle (Tba) ;
  - 4-[(4-chlorophényl)sulfonylaminocarbonyl]phénylcarbonyle (4-Cl-$\phi$-SAC-Bz) ;
  - 4-[(4-bromophényl)sulfonylaminocarbonyl]phénylcarbonyle (4-Br-$\phi$-SAC-Bz)
  - 4-[phénylsulfonylaminocarbonyl]phénylcarbonyle ($\phi$-SAC-Bz) ;
- $R_2$ est la chaîne latérale des aminoacides Phe, Tyr ou Leu ou est cyclohexylméthyle ;
- $R_3$ est la chaîne latérale des aminoacides Gly ou Ile ;
- X' est un amino-$\beta$-halogénoalkylène en $C_1$-$C_6$ dans lequel l'amino-$\beta$-halogénoalkylène en $C_1$-$C_6$ est $-CH_2CH_2CH(CHF_2)NH_2$, $-CH_2CF_2CH_2NH_2$, $-CH_2CH(CHF_2)CH_2NH_2$, $-CH_2CHFCHFCH_2NH_2$ ou bien est $R_4$ où $R_4$ est H, alkyle en $C_1$-$C_6$, phényle, benzyle, phénéthyle, cyclohexyle, cyclohexylméthyle ou 2-pyridylméthyle.

**2.** Composé selon la revendication 1, ledit composé étant
$P_g$-Phe-[$CF_2$GlyNH]$COCH_2C_6H_5$,
$P_g$-Phe-[$CF_2$GlyNH]$CO(CH_2)_3NH_2$,
$P_g$-Phe-[$CF_2$GlyNH]$COCH_2CF_2CH_2NH_2$ ou

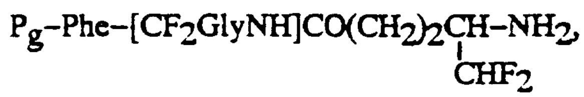

où $P_g$ est
4-[(4-chlorophényl]sulfonylaminocarbonyl]phénylcarbonyle (4-Cl-$\phi$-SAC-Bz),
4-[(4-bromophényl)sulfonylaminocarbonyl]phénylcarbonyle (4-Br-$\phi$-SAC-Bz) ou
4-[(phénylsulfonylaminocarbonyl]phénylcarbonyle ($\phi$-SAC-Bz).

**3.** Utilisation d'un composé de formule Iwb tel que défini dans la revendication 1 ou 2 pour la préparation d'une préparation pharmaceutique pour traiter le syndrome de l'immunodéficience acquise.

**4.** Composé selon la revendication 1 ou 2 ou mélange de celui-ci destiné à être utilisé comme composé pharmaceutiquement actif.

**Revendications pour l'Etat contractant suivant : ES**

**1.** Procédé pour la préparation des composés de formule :

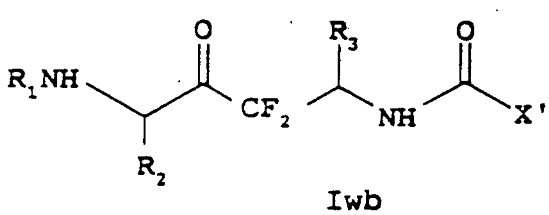

Iwb

et ses hydrates, isostères ou sels pharmaceutiquement acceptables, où

- $R_1$ est H, ou un groupe amino-protecteur choisi parmi :
  - isovaléryle (Iva) ;
  - t-butyloxycarbonyle (Boc) ;
  - carbobenzoxy (Cbz) ;
  - t-butylacétyle (Tba) ;
  - 4-[(4-chlorophényl)sulfonylaminocarbonyl]phénylcarbonyle (4-Cl-$\phi$-SAC-Bz) ;

4-[(4-bromophényl)sulfonylaminocarbonyl]phénylcarbonyle (4-Br-$\phi$-SAC-Bz)
4-[phénylsulfonylaminocarbonyl]phénylcarbonyle ($\phi$-SAC-Bz) ;

$R_2$ est la chaîne latérale des aminoacides Phe, Tyr ou Leu ou est cyclohexylméthyle ;

$R_3$ est la chaîne latérale des aminoacides Gly ou Ile ;

X' est un amino-$\beta$-halogénoalkylène en $C_1$-$C_6$ dans lequel l'amino-$\beta$-halogénoalkylène en $C_1$-$C_6$ est $-CH_2CH_2CH(CHF_2)NH_2$, $-CH_2CF_2CH_2NH_2$, $-CH_2CH(CHF_2)CH_2NH_2$, $-CH_2CHFCHFCH_2NH_2$ ou bien est $R_4$ où $R_4$ est H, alkyle en $C_1$-$C_6$, phényle, benzyle, phénéthyle, cyclohexyle, cyclohexylméthyle ou 2-pyridylméthyle

qui comprend :

1) le clivage de P'g d'un composé de formule XV

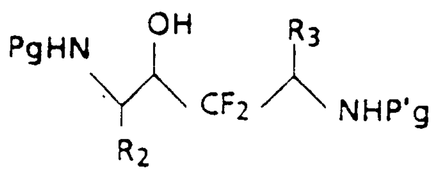

XV

dans laquelle $R_2$ et $R_3$ sont tels que définis ci-dessus et Pg et P'g sont chacun des groupes protecteurs ;

2) le couplage du composé résultant avec HOC(O)X' dans lequel X' est tel que défini ci-dessus pour donner le composé de formule XVI

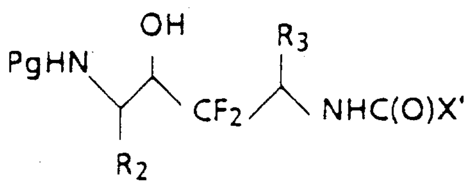

XVI

3) l'élimination éventuelle du groupe Pg de ce composé ;

4) le couplage avec $R'CO_2H$, $R'CO_2H$ étant l'équivalent de $R_1OH$ ;

5) l'oxydation du composé résultant ;

6) la déprotection éventuelle du composé résultant pour donner le composé de formule Iwb.

**2.** Procédé selon la revendication 1, dans lequel on fait réagir un composé de formule XV dans laquelle :

- le reste

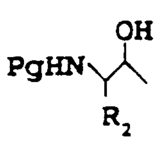

représente
Pg-Phe- dans lequel Pg est
4-[4-chlorophényl)sulfonylaminocarbonyl]phénylcarbonyl(4-Cl-$\phi$-SAC-Bz) ;
4-[4-bromophényl)sulfonylaminocarbonyl]phénylcarbonyl(4-Br-$\phi$-SAC-Bz) ou
4-[phénylsulfonylaminocarbonyl]phénylcarbonyl($\phi$-SAC-Bz) ;

- $R_3$ est la chaîne latérale de Gly ;

- P'g est tel que défini dans la revendication 1,

avec un composé de formule : HOC(O)X' dans laquelle X représente un groupe choisi parmi :
$-CH_2C_6H_5$,
$-(CH_2)_3NH_2$,
$-(CH_2)COCH_2CF_2CH_2NH_2$, ou

$$\begin{array}{l}-(CH_2)_2CH-NH_2\\ \qquad\quad\; CHF_2\end{array}$$

pour donner les composés suivants :
Pg-Phe-[$CF_2$GlyNH]$COCH_2C_6H_5$,
Pg-Phe-[$CF_2$GlyNH]$CO(CH_2)_3NH_2$,
Pg-Phe-[$CF_2$GlyNH]$COCH_2CF_2CH_2NH_2$, ou

$$\begin{array}{l}Pg-Phe-[CF_2GlyNH]CO(CH_2)_2\underset{|}{C}H-NH_2\\ \qquad\qquad\qquad\qquad\qquad\qquad\quad\; CHF_2\end{array}$$

dans lesquels Pg est tel que défini ci-dessus.

**3.** Utilisation d'un composé de formule Iwb tel que défini dans les revendications 1 ou 2 pour la préparation d'une composition pharmaceutique pour le traitement du syndrome de l'immunodéficience acquise.